# EUROPEAN PATENT APPLICATION

(11) **EP 4 112 633 A1**
(43) Date of publication of application: **04.01.2023**
(21) Application number: 21760612.8
(22) Date of filing: 02.03.2021
(51) Int. Cl.: C07K 14/005, A61K 39/25, A61K 47/68, A61P 25/00, A61K 39/00

(54) **VARICELLA ZOSTER VIRUS FUSION PROTEIN AND IMMUNOGENIC COMPOSITION COMPRISING SAME**

(30) Priority: 28.02.2020 KR 20200025608
(71) Applicant: Celltrion, Inc., Incheon 22014 (KR)
(72) Inventor: RYU, Dong Kyun, Incheon 22014 (KR); KIM, Pan Kyeom, Incheon 22014 (KR); NOH, Han Mi, Incheon 22014 (KR); PARK, Geun Soo, Incheon 22014 (KR); LEE, Soo Young, Incheon 22014 (KR); SHIM, Eun Yeong, Incheon 22014 (KR); KIM, Min Soo, Incheon 22014 (KR); LA, Wan Geun, Incheon 22014 (KR)
(74) Representative: Cabinet Laurent & Charras
(86) International application number: PCT/KR2021/002580
(87) International publication number: WO 2021/172971

(57) **Abstract**

The present invention relates to a Varicella zoster virus fusion protein and an immunogenic composition comprising same and, more specifically, to a fusion protein comprising the glycoprotein E (gE) of Varicella zoster virus (VZV) and a constant region of an immunoglobulin molecule, and an immunogenic composition comprising same. The present invention not only remarkably increases a Varicella zoster virus-specific cell-mediated immune response, but also exhibits the effect of rapidly and potently inducing an immune response and sustaining the immune response for a long period of time, compared to preexisting vaccines, thereby can be usefully used to prevent Varicella zoster-related diseases.

## Description

### Technical Field

The present invention relates to a varicella zoster virus fusion protein and an immunogenic composition including the same. More particularly, the present invention relates to a fusion protein including a glycoprotein E (gE) of varicella zoster virus (VZV) and a constant region of an immunoglobulin molecule and to an immunogenic composition including the same.

### Background Art

Varicella zoster virus (VZV) is a human alphaherpesvirus, which is a member of the genus varicellovirus, and causes varicella (chickenpox) and zoster (shingles). The primary infection with the varicella zoster virus occurs in epithelial cells of the upper respiratory mucosa and develops a chickenpox characterized by vesicular rash on the whole skin through a latency period of 10 to 21 days. During the primary infection, varicella zoster viruses can enter the sensory nerve cells of the host's ganglia by axonal transport from the skin or blood infected with the viruses, thereby causing latent infection for many years. Varicella zoster viruses latent in the ganglion may become reactive, causing shingles when the host has weak immunity. Shingles is known to be accompanied by severe pain and a bleb-type skin rash in ganglion-distributed dermatomes and is known to cause severe sequelae such as post-herpetic neuralgia (PHN) if not treated appropriately.

The genome of the varicella zoster virus genome has a linear double helix DNA structure with a length of about 125,000 bp and encodes at least 71 open reading frames (ORFs) and promoters. Nine of these constitute the surface glycoproteins (gB, gC, gE, gH, gI, gK, gL, gN, and gM) of the viral envelope and are known to be involved in viral replication and entry.

Currently used vaccines for herpes zoster prophylaxis largely include live attenuated vaccines or recombinant protein vaccines containing an immune enhancer. The live attenuated vaccine is the initial mode in which attenuated live viruses are, and the recombinant protein vaccine containing an immune enhancer is a mode in which a protein antigen includes an immune enhancer.

The method of using the recombinant protein vaccines containing an immune enhancer is intended to compensate for the disadvantages of existing live attenuated vaccines in a manner described below. The live attenuated vaccines exhibit relatively low efficacy in preventing herpes zoster and postherpetic neuralgia and shows greatly decreasing efficacy with increasing age. However, it has been confirmed that the recombinant protein vaccines with an immune enhancer exhibit high efficacy and protection regardless of age through clinical trials. In addition, the live attenuated vaccines have the disadvantage that they cannot be used for immunocompromised patients because they are still manufactured using live viruses even though the pathogenicity of the viruses is removed. However, the recombinant protein vaccines containing an immune enhancer have the advantage that they can be used even for those with weakened immune systems.

Such a recombinant protein vaccine containing an immune enhancer is an improved vaccine in terms of efficacy and safety of the live attenuated vaccine. However, the prevention rate of the vaccine against herpes zoster is about 90%, and the vaccine may cause local pain because the vaccine includes a saponin-based immune enhancer. In addition, for the prevention of herpes zoster and postherpetic neuralgia, it is known that the activation of cell-mediated immunity (CMI) as well as the induction of a humoral immune response to the varicella zoster virus antigen play a direct role.

Accordingly, there is a need for the development of a new herpes zoster vaccine composition that does not have the disadvantages of the live attenuated vaccines and the immune enhancer-containing recombinant protein vaccines, which is safer, and which can selectively increase cell-mediated immunogenicity.

For example, Zostavax, a herpes zoster vaccine that is currently widely inoculated around the world, is a representative live attenuated vaccine and is manufactured through animal cell culture and purification processes using a vaccine virus strain called OKA. This type of vaccine has a low efficacy, and in particular, the efficacy drops sharply with age. This type of vaccine cannot be used for immunocompromised patients. In addition, Shingrix, a widely used herpes zoster vaccine, is a representative recombinant vaccine containing an immune enhancer, in which and gE glycoprotein is produced through animal cell culture and purification process, and AS01 immune enhancers (including liposome, MPL, and QS21) are added. The efficacy is approximately 90% and side effects such as local pain attributable to an immune enhancer are reported.

Since the conventional vaccines described above were problematic in efficacy and safety, the effectiveness of prophylaxis is somewhat limited, and patients experience local pain.

### Disclosure

### Technical Problem

Accordingly, the inventors evaluated the efficacy of a new vaccine composition and as a result have confirmed that the new vaccine exhibited greatly improved efficacy and reduced side effects, thereby having completed the present invention.

One problem to be solved by the present invention is to provide a fusion protein comprising a glycoprotein E (gE) of a varicella zoster virus (VZV) and a constant region of an immunoglobulin molecule.

Another problem to be solved by the present invention is to provide a nucleic acid molecule encoding the fusion protein.

A further problem to be solved by the present invention is to provide an expression vector comprising the nucleic acid molecule.

A yet further problem to be solved by the present invention is to provide a host cell into which the expression vector is transformed.

In addition, a yet further problem to be solved by the present invention is to provide a method of preparing the fusion protein.

In addition, a yet further problem to be solved by the present invention is to provide an immunogenic composition comprising the fusion protein.

In addition, a yet further problem to be solved by the present invention is to provide a kit comprising the immunogenic composition

In addition, a yet further problem to be solved by the present invention is to provide a method of inducing an immune response by administering the immunogenic composition.

In addition, a yet further problem to be solved by the present invention is to provide a method of preventing varicella zoster or postherpetic neuralgia by administering the immunogenic composition.

### Technical Solution

In order to solve the above problems, the present invention provides a fusion protein comprising a glycoprotein E (gE) of a varicella zoster virus and a constant region of an immunoglobulin molecule.

In the fusion protein according to the present invention, the glycoprotein E of the varicella zoster virus may be truncated such that a C-terminus anchor region is removed. The glycoprotein E of the fusion protein according to the present invention may be represented by SEQ ID NO: 1.

In the fusion protein according to the present invention, the constant region (e.g., Fc site) of the immunoglobulin molecule may enhance immunogenicity by binding with an Fc receptor. The constant region of the immunoglobulin molecule of the fusion protein according to the present invention may be a constant site of an immunoglobulin heavy chain but is not limited thereto. The immunoglobulin may be any one selected from the group consisting of IgG, IgM, IgA, IgD, and IgA but is not limited thereto. In one embodiment, the immunoglobulin may be IgG but is not limited thereto. In one embodiment, the IgG may be any one selected from the group consisting of IgG1, IgG2, IgG3, and IgG4 but is not limited thereto. In one embodiment, the constant region of the immunoglobulin molecule may be a constant region of an IgG1 heavy chain but is not limited thereto. In one embodiment, the constant region of the immunoglobulin molecule may include a hinge region, a CH2 domain and a CH3 domain of IgG1. In another embodiment, the constant region of the immunoglobulin molecule may further include a CH1 domain of IgG1 but is not limited thereto. In one embodiment, the constant region of the immunoglobulin molecule may include an Fc site. In one embodiment, the Fc site may bind with an Fc receptor to enhance immunogenicity.

In one embodiment, the Fc site may include an Fc variant. In one embodiment, the Fc variant may include one or more Fc variations capable of enhancing binding with Fc receptors. In one embodiment, the Fc variant may include one or more Fc variations capable of enhancing binding with Fc receptors to enhance immunogenicity. In one embodiment, the Fc variant may include one or more variations selected from the group consisting of G236A, S239D, A330L, and I332E. In one embodiment, the Fc variant may include all of the G236A, S239D, A330L, and I332E variations but is not limited thereto. In one embodiment, the Fc site may include the sequence of SEQ ID NO: 2 or SEQ ID NO: 3 but is not limited thereto. In one embodiment, the Fc variant may include the sequence of SEQ ID NO: 4 or SEQ ID NO: 5 but is not limited thereto. In one embodiment of the present invention, an Fc of a mouse or an Fc variant of a mouse was used for animal experiments, but the ordinarily skilled in the art can use a human Fc or a human Fc variant instead of the Fc or Fc variant of a mouse to accomplish the objectives of the present invention.

In addition, the present invention provides a nucleic acid molecule encoding the fusion protein.

In addition, the present invention provides an expression vector comprising the nucleic acid molecule.

In addition, the present invention provides a host cell into which the expression vector is transformed.

In addition, the present invention provides a method of preparing a fusion protein,
the method comprising:
i) introducing the expression vector into an animal cell expression system; and
ii) performing expression of the fusion protein.

In order to accomplish another objective of the present invention, the present invention provides an immunogenic composition comprising the fusion protein described above.

In one embodiment, the fusion protein may be a monomer but is not limited thereto. In one other embodiment, the fusion protein may be a dimer but is not limited thereto. Herein, the dimer may be made by a disulfide bond in a hinge region between the two fusion proteins but may not be limited thereto.

The immunogenic composition according to the present invention may additionally include an adjuvant, in which the adjuvant may be one or more selected from the group consisting of, but not limited to, toll-like receptor (TLR4) agonists, aluminum salts saponins, and liposomes.

The TLR4 agonists may be any one or more selected from the group consisting of monophosphoryl lipid A (MPL) and 3D-MPL but are not limited thereto.

The aluminum salts may be any one or more selected from the group consisting of aluminum hydroxide, aluminum phosphate, and aluminum sulphate but are not limited thereto.

The saponins may be any one or more selected from the group consisting of QS21, QS17, and QuilA, but are not limited thereto.

In addition, in order to accomplish a further objective of the present invention, there is provided a kit comprising the immunogenic composition described above.

In addition, in order to accomplish a yet further objective of the present invention, there is provided a method of inducing an immune response by administering the immunogenic composition.

In addition, in order to accomplish a yet further objective of the present invention, there is provided a method of preventing varicella zoster or postherpetic neuralgia by administering the immunogenic composition.

### Advantageous Effects

The fusion protein comprising a glycoprotein E (gE) of a varicella zoster virus and a constant region of an immunoglobulin molecule, according to the invention, and the immunogenic composition comprising the same fusion protein can significantly increase the varicella zoster virus-specific cell-mediated immune response. Accordingly, the fusion protein according to the present invention not only exhibits a more rapid and stronger immune response than existing vaccines but also exhibits a long-lasting effect. Therefore, the fusion protein can be usefully used to prevent varicella zoster-related diseases.

### Description of Drawings

FIG. 1 shows the number of immune cells secreting interferon gamma specifically for a varicella zoster virus antigen that is a combination of gE-Fc + MPL + alum.
FIG. 2 shows the production amount of IgG1 and IgG2a antibodies specific for the surface glycoprotein E of varicella zoster virus, which is a combination of gE-Fc + MPL + alum.
FIG. 3 shows the number of immune cells secreting interferon gamma specifically for a varicella zoster virus antigen which is a combination of gE-Fc' + MPL + alum.
FIG. 4 shows the production amount of IgG1 and IgG2a antibodies specific for surface glycoprotein E of varicella zoster virus, which is a combination of gE-Fc' + MPL + alum.

### Best Mode

Hereinafter, the present invention will be described in detail. However, the present disclosure is intended to assist in the understanding of the present invention, and the scope of the present invention is not limited in any sense to the matters described in the detailed description.

The scope of the present invention is not limited by the following description, and in particular, the scope of the present invention may include anything that is variable according to the experimental conditions described in Examples and the like below. The scope of the present invention will be defined by the appended claims, and therefore the terminology used herein for understanding is for the purpose of describing detailed embodiments of the present invention, but the scope of the invention should not be limited thereto.

Unless otherwise defined herein, all technical and scientific terms used herein are to be construed in the same sense as commonly understood by the ordinarily skill in the art. All documents referenced herein are incorporated herein by reference in their entirety for the purpose of describing the present invention.

The present invention relates to a fusion protein including a glycoprotein E (gE) of a varicella zoster virus (VZV) and a constant region of an immunoglobulin molecule. More specifically, the present invention provides an Fc-fusion protein in which the amino terminus of the fragment crystallizable (Fc) region of the immunoglobulin heavy chain is linked, via a peptide bond, to the carboxyl terminus of the surface glycoprotein E of the varicella zoster virus.

The glycoprotein E of the varicella zoster virus may be truncated such that a C-terminal anchor region is removed. In this case, the glycoprotein E may be represented by the sequence of SEQ ID NO: 1.

The immunoglobulin may be any one selected from the group consisting of IgG, IgM, IgA, IgD, and IgA but may not be limited thereto. In one embodiment, the immunoglobulin may be IgG but may not be limited thereto. In one embodiment, the IgG may be any one selected from the group consisting of IgG1, IgG2, IgG3, and IgG4 but is not limited thereto. In one embodiment, the constant region of the immunoglobulin molecule may be, but may not be limited to, a constant region of an IgG1 heavy chain. In one embodiment, the constant region of the immunoglobulin molecule may include a hinge region, a CH2 domain, and a CH3 domain of IgG1. The constant region of the immunoglobulin molecule may further include, but is not limited to, a CH1 domain of IgG1. In one embodiment, the constant region of the immunoglobulin molecule may be an Fc site but may not be limited thereto. In one embodiment, the Fc site may bind with an Fc receptor to enhance immunogenicity but is not limited thereto. In one embodiment, the Fc site may include, but is not limited to, the sequence of SEQ ID NO: 2 or SEQ ID NO: 3.

In one embodiment, the Fc site may include, but is not limited to, an Fc variant. In one embodiment, the Fc variant may include one or more variations selected from the group consisting of G236A, S239D, A330L, and I332E but is not limited thereto. In one embodiment, the Fc variant may include, but is not be limited to, all of the G236A, S239D, A330L, and I332E variations. The numbering scheme of the Fc variant positions follows the EU scheme but is not limited thereto. In one embodiment, the Fc variant may include, but is not be limited to, the sequence of SEQ ID NO: 4 or SEQ ID NO: 5.

In addition, the present invention provides a nucleic acid molecule encoding the fusion protein.

In addition, the present invention provides an expression vector including the nucleic acid molecule.

In addition, the present invention provides a host cell into which the expression vector is transformed.

In addition, the present invention provides a method of preparing a fusion protein,
the method comprising:
i) introducing the expression vector into an animal cell expression system; and
ii) performing expression of the fusion protein.

In addition, the present invention provides an immunogenic composition including the fusion protein.

In one embodiment, the fusion protein may be a monomer but is not limited thereto. In one other embodiment, the fusion protein may be a dimer but is not limited thereto. Herein, the dimer may be made by a disulfide bond in a hinge region between the two fusion proteins but is not limited thereto.

The immunogenic composition according to the present invention may additionally include an adjuvant, in which the adjuvant may be at least one selected from the group consisting of, but not limited to, toll-like receptor (TLR4) agonists, aluminum salts saponins, and liposomes.

The TLR4 agonists may be one or more selected from the group consisting of monophosphoryl lipid A (MPL) and 3D-MPL but are not limited thereto.

The aluminum salts may be one or more selected from the group consisting of aluminum hydroxide, aluminum phosphate, and aluminum sulphate but are not limited thereto.

The saponin is a triperten glycosidic material widely distributed in the plant and marine animal world and is an adjuvant largely accepted and approved in the art. According to one embodiment of the present invention, the saponin may be any one or more selected from the group consisting of, but is not limited to, QS21, QS17, and QuilA. According to another embodiment of the present invention, the saponin may be, but is not limited to, preferably QS21. QS21 is obtained through HPLC purification of extracts derived from the shell of Quillaja saponaria and is expressed as acylated 3, 28-bisdesmodic triterpene glycosides (1,3).

The liposome is a lipid-based oval construct formed of a phospholipid bilayer and is typically included in a vaccine composition or a pharmaceutical composition, etc., to serve as a drug carrier. According to one embodiment of the present invention, the liposomes may be one or more selected from the group consisting of, but is not limited to, dimethyldioctadecylammonium bromide (DDA), 1,2-dioleoyl-3-trimethyl ammonium propane (DOTAP), 3β-[N-(N,N-dimethylaminoethane) carbamoyl cholesterol (DC-Chol), 1,2-dioleoyloxy-3-dimethylammonium propane (DODAP), 1,2-di-O-octadecenyl-3-triethylammonium propane (DOTMA), 1,2-dimyristoleoyl-sn-glycero-3-ethylphosphocholine (14:1 ethyl PC), 1-palmitoyl-2-oleoyl-sn-glycero-3-ethylphosphocholine (16:0-18:1 ethyl PC), 1,2-dioleoyl-sn-glycero-3-ethylphosphocholine (18:1 ethyl PC), 1,2-distearoyl-sn-glycero-3-ethylphosphocholin (18:0 ethyl PC), 1,2-dipalmitoyl-sn-glycero-3-ethylphosphocholine (16:0 ethyl PC), 1,2-dimyristoyl-sn-glycero-3-ethylphosphocholine (14:0 ethyl PC), 1,2-dilauroyl-sn-glycero-3-ethylphosphocholin (12:0 ethyl PC), N1-[2-((1S)-1-[(3-aminopropyl)amino]-4-[di(3-amino-propyl) amino]butylcarboxamido)ethyl]-3,4- di[oleyloxy]-benzamide (MVL5), 1,2-dimyristoyl-3-dimethylammonium-propane (14:0 DAP), 1,2-dipalmitoyl-3-dimethyl ammonium-propane (16:0 DAP), 1,2-distearoyl-3-dimethylammonium-propane (18:0 DAP), N-(4-carboxybenzyl)-N,N-dimethyl-2,3- bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TA), 1,2-dimyristoyl-3-trimethyl ammonium-propane (14:0 TAP), and N4-Cholesteryl-Spermine (GL67).

In addition, the liposomes may additionally include to one or more neutral lipids selected from the group consisting of [0020] 1,2-dimyristoyl-snglycero-3-phosphorylcholine (DMPC), 1,2-dioleoyl-sn-glycero-3-phosphocholine (DOPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphoethanolamine (PE), phosphatidylglycerol (PG), phosphoric acid (PA), and phosphatidylcholine (PC), but are not limited thereto.

In addition, the present invention provides a kit including the immunogenic composition.

In addition, in order to accomplish a yet further objective of the present invention, there is provided a method of inducing an immune response by administering the immunogenic composition.

In addition, in order to accomplish a yet further objective of the present invention, there is provided a method of preventing varicella zoster or postherpetic neuralgia by administering the immunogenic composition.

Each of the features described herein may be used in combination, and the fact that each of the features is recited in different subordinate claims of the appended claims does not imply that they cannot be used in combination.

### Example 1. Method of preparing fusion protein of viral antigen and Fc site

First, an gE-Fc fusion protein was prepared in a manner described below.

DNA of a surface glycoprotein E (gE, antigen) of the varicella zoster virus was synthesized and cloned into the pCT146 vector, which is an animal expression vector. The synthesized gE DNA was cleaved with two restriction enzymes, *Nhe*I and *Pme*I, and the pCT146 vector was cleaved with the same restriction enzymes. The cleaved gE and vector DNAs were linked by the T4 ligase. The cloned pCT146 DNA was transformed into *E.coli* and the colonies were analyzed to select for bacterial clones in which the gE gene was inserted into the pCT146 vector. This vector was named pCT430.

Cloning to remove the stop codon of the gE gene of pCT430 was first performed to prepare a gE-Fc fusion protein. The gE gene of the pCT430 vector was amplified by polymerase chain reaction (PCR) technology using a forward primer including a Nhe I recognition sequence and a reverse primer that includes a Pme I recognition sequence and from which a stop codon is removed. Using the *Nhe*I and *Pme*I restriction enzymes, a pCT430 vector that is cloned with the gE DNA and from which the stop codon was removed was prepared.

Each of the Fc gene sequence of human IgG1 and the Fc gene sequence of mouse (rat) IgG2a was amplified with polymerase chain reaction technology using forward, reverse primers each including the recognition sequence of the restriction enzyme Pme I and cleaved with the restriction enzyme Pme I. It is well known in the art that the Fc of the mouse (rat) IgG2a functionally corresponds with the Fc of the human IgG1 (Falk Nimmerjahn et al. Nat Rev Immunol. 2008 Jan;8(1):34-47). In the description herein, the Fc amino acid sequence of the human IgG1 and the Fc amino acid sequence of the mouse IgG2a were designated SEQ ID NO: 2 and SEQ ID NO: 3, respectively.

The pCT430 vector from which the stop codon of the gE gene was removed was cleaved with the same restriction enzyme, and then DNA ligation was performed in the same manner as above to obtain vectors in which the Fc gene of the antibody was inserted into the carboxyl terminus of the gE gene. The vectors were named pCT486 and pCT487, respectively.

Next, the selected colonies were cultured to purify the DNA. CHO-K1 animal cells belonging to one of the Chinese hamster ovary cell lines (CHO) was transformed with the purified high-purity DNA. Mmethotrexate (MTX), which is an inhibitor of dihydrofolate reductase (DHFR) serving as a selection marker was added to the CHO-K1 cells, and the transformed cell lines were selected through subculture. The gE-Fc protein expelled out of the cell was purified with Protein A affinity chromatography and size exclusion chromatography using protein size differences.

### Example 2. Preparation of MPL and aluminum hydroxide

Next, for assay of the immunogenic composition of the present invention, MPL and aluminum hydroxide as adjuvants (immune enhancers) were prepared in a way described below.

As monophosphoryl Lipid A (also known as MPL or MPLA), MPLA-SM VacciGrade^{™} (Catalog code: vac-mpla) which is a product of InvivoGen Limited was used. The MPL was dispensed with dimethyl sulfoxide (DMSO) according to the manufacturer's instructions and stored frozen at -20°C.

MPL is extracted from lipopolysaccharide (LPS) produced from Re mutant of *Salmonella Minnesota* R595 strain. The lipid A is primarily involved in the endotoxic activity of LPS. MPL is a substance obtained by eliminating one phosphate group from the lipid A. MPL exhibits reduced toxicity and a similar level of immunoenhancing effect when compared to the Lipid A (refer to (Infect Immun. 71 (5): 2498-507, Int Immunol. 14(11):1325-32, J. Biol. Chem. 257(19): 11808-15, Infect Immun. 79(9): 3576-3587). Like LPS, MPL functions as a toll-like receptor 4 (TLR4 agonist) and is also known to potently elicit Th1 immune responses (Science 316 (5831): 1628-32, Infect Immun. 75 (12): 5939-46).

Next, Alhydrogel^{®} adjuvant 2%, catlog# vac-alu-250, which is a product from InvivoGen Limited, was dispensed for use as aluminum hydroxide.

Aluminum-based adjuvants are known to increase antigen uptake of antigen presenting cells (APCs), to elicit a Th2 immune response, and not to elicit a Th1 immune response (Immunity 33(4): 492-503).

The composition of the invention was prepared by mixing the MPL and the aluminum hydroxide with the gE-Fc fusion protein prepared in Example 1, the concentration of the mixture was adjusted with a phosphate-buffered saline (PBS) solution.

### Experimental Example 1 ELISPOT Assay

To evaluate the efficacy of the composition prepared in Example 2 described above, an experiment was conducted in a manner described below.

Since herpes zoster has a pathological characteristic that it develops, in people with weakened immunity, by the reactivation of the varicella zoster virus, which has been dormant in the ganglia through chickenpox infection, in order to mimic chickenpox infection in animals, a live attenuated vaccine was heat inactivated, and then priming immunization was performed by subcutaneously injecting the vaccine once into female BALb/c mice (rats).

In about 5 weeks (36 days) after the priming immunization, compositions of various conditions comprising an adjuvant and a fusion protein antigen in which the varicella zoster virus surface glycoprotein E (gE) and the Fc site of a mouse (rat) IgG2a antibody are bound, were immunized by intramuscular injection twice at 4-week intervals. Whole blood was drawn from the postcaval vein of the mice in about 4 weeks (28 days) after secondary immunization to measure the VZV-specific humoral and cellular immunity elicited by the vaccine compositions, and peripheral blood mononuclear cells (PBMCs) were isolated.

The experimental designs for evaluating the cell-mediated immune response of the compositions are shown in Table 1 below. The term "gE-Fc" in Table 1 refers to the antigen in which the Fc site of the mouse IgG2a antibody is fused with the glycoprotein E (gE) of the varicella zoster virus (VZV), the term "MPL" refers to monophosphoryl lipid A serving as an adjuvant, and the term "alum" refers to aluminum hydroxide (see Examples 1 and 2). In addition, "Shingrix" is a recombinant varicella zoster vaccine (manufactured by GSK). It is a commercialized vaccine (liposome-based gE + MPL + QS21 combination) containing the surface glycoprotein E of a liposome-based varicella zoster virus, MPL, and QS21. In addition, the final volume of each composition in Groups 1 to 4 was 100 µl which was administered.

**[Table 1]**

| Group | Vaccine composition | Priming inoculation Day (0) | Primary immunization (5 weeks) | Secondary immunization (9 weeks) | Sample collection (13 weeks) |
|---|---|---|---|---|---|
| 1 | PBS | SC | IM | IM | Serum, PBMC |
| 2 | Shingrix | SC | IM | IM | Serum, PBMC |
| 3 | gE-Fc (30 µg)+ MPL + alum | SC | IM | IM | Serum, PBMC |
| 4 | gE-Fc (90 µg)+ MPL + alum | SC | IM | IM | Serum, PBMC |

To assess the cellular immune response of each composition, an Interferon gamma (IFNγ) enzyme-linked immunospot (ELISPOT) assay was performed using isolated peripheral blood mononuclear cells in 4 weeks after secondary immunization. To this end, remaining red blood cells (RBCs) were removed from the mice PBMC samples using a red blood cell (RBC) lysis buffer. The RBC-removed samples were treated by reacting prepared lymphocytes with a varicella zoster antigen (Microbix Biosystems Inc., Catalog number EL-03-02) or Concanavalin A (Sigma, Catalog number C5275) with the use of Mouse IFN-γELISPOT plus (MABTECH, Catalog number 3321-4AST). The assay was performed by detecting interferon gamma proteins secreted from the lymphocytes and counting spot forming cells (SFCs).

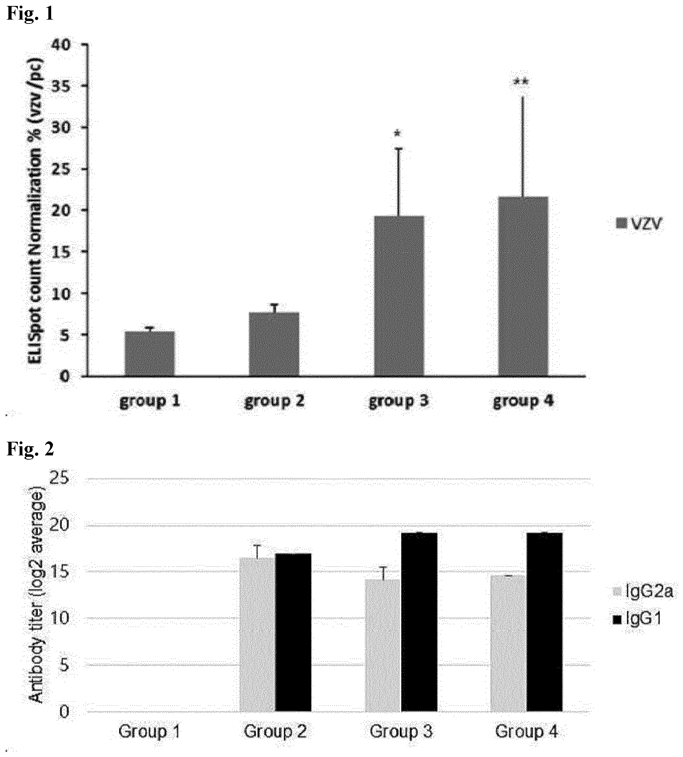

The activity of each group of lymphocytes was normalized with the activity of cell-mediated immune response elicited by Concanavalin A, which was a positive control (PC), and the cell-mediated immune response of the VZV-specific lymphocytes is shown in FIG. 1. VZV in FIG. 1 designates the VZV-specific cell-mediated immune response, in which ^{∗} or ^{∗∗} indicates a case where a p value is less than (p value < 0.05) for group 1, which means there is a statistically significant difference.

As a result, it was confirmed that the composition including the gE-Fc fusion protein and MPL/alum adjuvants elicited a statistically significant and remarkably improved VZV-specific cell-mediated immune response when compared to the PBS (negative control), and also elicited an equivalent or greater cellular immune response than the Singrix vaccine (see FIG. 1).

As shown in FIG. 1, the gE-Fc + MPL + alum combination showed a higher level of cellular immune response at high concentrations (for example, 90 µg) than at low concentrations (for example, 30 µg).

### Experimental Example 2 ELISA Assay

To evaluate the efficacy of each of the compositions (Groups 1-4) of the combinations of Experimental Example 1, an additional experiment was conducted in a manner described below.

In approximately 4 weeks after secondary immunization, the amount of IgG1 antibody and IgG2a antibody elicited by the vaccine composition was measured by ELISA assay from the serum isolated from the mouse's blood.

The VZV gE antigen (2 µg/ml) was coated on a 96-well plate, using a coating solution (also called coating buffer or carbonate/bicarbonate buffer, manufactured by Sigma, catalog number C3041-100CAP) at 4°C for 16 to 18 hours. Washing was performed with a diluent (Teknova, catalog# 2D5120-1L; 1% BSA, 0.05% Tween-20, IX PBS) three times and the plate was blocked with the diluent at room temperature for 1 hour. After three times of washing, the prepared serum sample was placed in the plate and reacted at room temperature for 1 hour 30 minutes to 2 hours. After 3 times of washing, the detected antibody, which is i) horseradish peroxidase-conjugated goat anti-mouse IgG1 (BioRad, Catalog# STAR132P) or ii) horseradish peroxidase-conjugated goat anti-mouse IgG2a (BioRad, Catalog# STAR133P), was added and reacted at room temperature for 1 hour. After 6 times of washing, 3,3', 5,5'-tetramethylbenzidine (TMB, manufactured by Sigma, Catalog# T0440) was placed in a plate and reacted for 5 minutes, and a sulfuric acid solution (H2SO4, Merck, Catalog# 109072) was added to stop the reaction. The absorbance (optical density) of each sample was measured with a plate reader. The results of those tests are shown in Tables 2 and 3 and FIG. 2. The results are measurements of the production amount of VZV surface glycoprotein E (gE), i.e., the VZV-specific IgG1 and IgG2a antibodies. The IgG1 production refers to the product of a humoral immune response, whereas the IgG2a production refers to the product of a cellular immune response.

As a result, as shown in Table 2, it was confirmed that the combination of gE-Fc + MPL + alum exhibited a better effect of producing the gE-specific IgG2a antibody than the PBS, indicating an increase in cellular immune response. In addition, as shown in Table 3, it was confirmed that the combination of gE-Fc + MPL + alum exhibited a superior effect of producing the gE-specific IgG1 antibody to the PBS, indicating an increase in humoral immune response (see FIG. 2).

In addition, as previously confirmed from FIG. 1, it was confirmed that the combination of gE-Fc + MPL + alum had a similar level of ability to elicit humoral and cellular immune responses when compared to the Singrix vaccine.

Accordingly, it was finally confirmed that the compositions of the present invention remarkably increased IgG1 and IgG2a antibody production when compared to the negative control, and it was finally confirmed that the compositions of the present invention elicited a similar level of cellular and humoral immune responses when compared to the Singrix vaccine (Table 2 and Table 3).

**[Table 2]**

| Gr oup | Vaccine composition | IgG2a antibody | | | Avera ge | Fold | Log2 average | Log2 Standard deviation |
|---|---|---|---|---|---|---|---|---|
| 1 | PBS | 0 | 0 | 0 | N/A | N/A | N/A | N/A |
| 2 | Shingrix | 12500 0 | 12500 0 | 2500 0 | 91667 | 5.0 | 16.5 | 1.3 |
| 3 | gE-Fc (30 µg)+ MPL + alum | 25000 | 25000 | 5000 | 18333 | 1.0 | 14.2 | 1.3 |
| 4 | gE-Fc (90 µg)+ MPL + alum | 25000 | 25000 | 2500 0 | 25000 | 1.4 | 14.6 | 0.0 |

**[Table 3]**

| Gr oup | Vaccine composition | IgG1 antibody | | | Avera ge | Fold | Log2 average | Log2 standard deviation |
|---|---|---|---|---|---|---|---|---|
| 1 | PBS | 0 | 0 | 0 | N/A | N/A | N/A | N/A |
| 2 | Shingrix | 12500 0 | 12500 0 | 12500 0 | 12500 0 | 1.0 | 16.9 | 0.0 |
| 3 | gE-Fc (30 µg)+ MPL + alum | 62500 0 | 62500 0 | 62500 0 | 62500 0 | 5.0 | 19.3 | 0.0 |
| 4 | gE-Fc (90 µg)+ MPL + alum | 62500 0 | 62500 0 | 62500 0 | 62500 0 | 5.0 | 19.3 | 0.0 |

### Example 3. Method of preparing fusion mutation protein of viral antigen and Fc site

First, an gE-Fc fusion mutation protein (gE-Fc') was prepared by a method described below.

DNA of a fusion protein in which the surface glycoprotein E (gE, antigen) of the varicella zoster virus and an Fc (G236A/S239D/A330L/I332E) mutation site of a mouse (rat) IgG2a was synthesized and cloned into a pCT146 vector, which is an animal expression vector. The synthesized gE-Fc' DNA was cleaved with two restriction enzymes, Nhe I and Pme I, and the pCT146 vector was cleaved with the same restriction enzymes. The cleaved gE-Fc' DNA and the cleaved vector were linked by the T4 ligase. The cloned pCT146 DNA was transformed into E.coli, and the colonies were analyzed to select bacterial clones with the PCT146 vector in which the gE gene was inserted. This vector was named pCT664. The amino acid sequence of the Fc (G236A/S239D/A330L/I332E) variant of the mouse IgG2a used in the present example was designated SEQ ID NO: 5. The amino acid sequence of the Fc (G236A/S239D/A330L/I332E) variant of the human IgG1 that was not used in the present example but can be used in other embodiments of the present invention was designated SEQ ID NO: 4.

Next, the selected colonies were cultured to purify the DNA. Transient transfection was performed with high-purity DNA, on an ExpiCHO-S animal cell which is one of the Chinese hamster ovary cell lines (CHO). The ExpiCHO-S cells were cultured by a fed-batch culturing method to produce the gE-Fc' fusion protein. The gE-Fc' protein expelled out of the cell was purified with the use of a protein A affinity chromatography.

### Experimental Example 3 ELISPOT Assay

To evaluate the efficacy of the compositions prepared in Example 2 and Example 3 described above, an experiment was conducted in a manner described below.

The priming immunization was performed by subcutaneously injecting a live attenuated vaccine once into C57BL/6 female mice (rats) to mimic chickenpox infection in animals.

In about 5 weeks (36 days) after the priming immunization, compositions diversely controlled to include an adjuvant and a fusion protein antigen in which the varicella zoster virus surface glycoprotein E (gE) and the Fc site of a mouse (rat) IgG2a antibody are bound were administered twice at 4-week intervals by intramuscular injection for immunization. Whole blood was drawn from the postcaval vein of the mice about 4 weeks (28 days) after secondary immunization to measure the VZV-specific humoral and cellular immunity elicited by the vaccine compositions, and peripheral blood mononuclear cells (PBMCs) were isolated.

The experimental designs for evaluating the cell-mediated immune response of the compositions are shown in Table 4 below. The term "gE-Fc" in Table 1 refers to the antigen in which the Fc site of the mouse IgG2a antibody is fused with the glycoprotein E (gE) of the varicella zoster virus, the term "MPL" refers to monophosphoryl lipid A serving as an adjuvant, and the term "alum" refers to aluminum hydroxide (see Examples 1, 1 and 3). In addition, "Shingrix" is a recombinant varicella zoster vaccine (manufactured by GSK). It is a commercialized vaccine (liposome-based gE + MPL + QS21 combination) containing the surface glycoprotein E of a liposome-based varicella zoster virus, MPL, and QS21. In addition, the final volume of each composition in Groups 1 to 4 was 50 µl which was administered.

**[Table 4]**

| Group | Vaccine composition | Priming inoculation Day (0) | Primary immunization (5 weeks) | Secondary immunization (9 weeks) | Sample collection (13 weeks) |
|---|---|---|---|---|---|
| 1 | PBS | SC | IM | IM | Serum, PBMC |
| 2 | Shingrix | SC | IM | IM | Serum, PBMC |
| 3 | gE-Fc (10 µg)+ MPL + alum | SC | IM | IM | Serum, PBMC |
| 4 | gE-Fc' (10 µg)+ MPL + alum | SC | IM | IM | Serum, PBMC |

To assess the cellular immune response of each composition, an Interferon gamma (IFNγ) enzyme-linked immunospot (ELISPOT) assay was performed using isolated peripheral blood mononuclear cells in 4 weeks after secondary immunization.

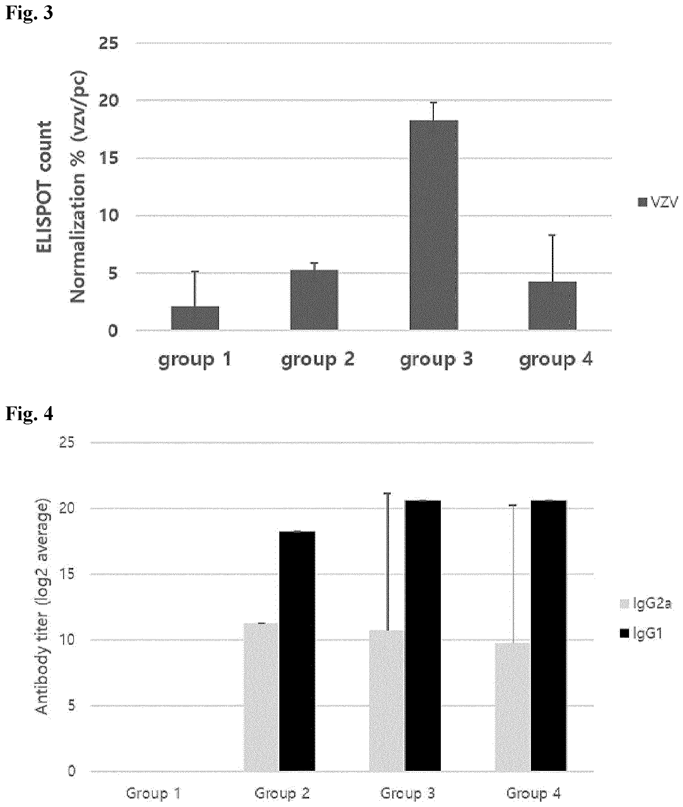

The activity of each group of lymphocytes was normalized with the activity of cell-mediated immune response elicited by Concanavalin A, which was a positive control (PC), and the cell-mediated immune response of the VZV-specific lymphocytes is shown in FIG. 3. In FIG. 3, VZV represents VZV-specific cell-mediated immune responses.

As a result, it was confirmed that the composition including the gE-Fc fusion protein and MPL/alum adjuvants elicited a remarkably improved VZV-specific cell-mediated immune response when compared to the PBS (negative control), and also elicited an equivalent or greater cellular immune response than the Singrix vaccine (see FIG. 3). Likewise, it was confirmed that the composition including the gE-Fc' fusion protein and MPL/alum adjuvants elicited a statistically significant and remarkably improved VZV-specific cell-mediated immune response when compared to the PBS (negative control), and also elicited an equivalent or greater cellular immune response than the Singrix vaccine (see FIG. 3).

### Experimental Example 4 ELISA Assay

To evaluate the efficacy of each of the compositions (Groups 1-4) of the combinations of Experimental Example 3, an additional experiment was conducted in a manner described below.

In approximately 4 weeks after secondary immunization, the amount of IgG1 antibody and IgG2a antibody induced by the vaccine composition was measured by ELISA assay from the serum isolated from the mouse's blood.

As a result, as shown in Table 5, it was confirmed that the combination of gE-Fc + MPL + alum exhibited a better effect of producing the gE-specific IgG2a antibody than the PBS, indicating an increase in cellular immune response. In addition, as shown in Table 6, it was confirmed that the combination of gE-Fc + MPL + alum exhibited a better effect of producing the gE-specific IgG1 antibody than the PBS, indicating an increase in humoral immune response (see FIG. 4).

In addition, as previously confirmed from FIG. 3, it was finally confirmed that the combination of gE-Fc + MPL + alum had a better ability to elicit humoral and cellular immune responses than the Singrix vaccine.

In addition, as shown in Table 5, it was confirmed that the combination of gE-Fc + MPL + alum exhibited a better effect of producing the gE-specific IgG2a antibody than the PBS, indicating an increase in cellular immune response. In addition, as shown in Table 6, it was confirmed that the combination of gE-Fc + MPL + alum exhibited a better effect of producing the gE-specific IgG1 antibody than the PBS, indicating an increase in humoral immune response (see FIG. 4).

In addition, as previously confirmed from FIG. 3, it was finally confirmed that the combination of gE-Fc' + MPL + alum had a similar level of ability to elicit humoral and cellular immune responses when compared to the Singrix vaccine.

Accordingly, it was finally confirmed that the compositions of the present invention remarkably increased IgG1 and IgG2a antibody production when compared to the negative control, and it was finally confirmed that the compositions of the present invention elicited a similar level of cellular and humoral immune responses when compared to the Singrix vaccine (refer to Tables 5 and 6).

**[Table 5]**

| Gr oup | Vaccine composition | IgG2a antibody | | | Averag e | Fold | Log2 averag e | Log2 standard deviation |
|---|---|---|---|---|---|---|---|---|
| 1 | PBS | 0 | 0 | 0 | N/A | N/A | N/A | N/A |
| 2 | Shingrix | 2500 | 2500 | 2500 | 2500 | 1.5 | 11.3 | 0.0 |
| 3 | gE-Fc (10 µg)+ MPL + alum | 2500 | 2500 | 100 | 1700 | 1.0 | 10.7 | 10.4 |
| 4 | gE-Fc'(10 µg)+ MPL + alum | 100 | 2500 | 0 | 867 | 0.5 | 9.8 | 10.5 |

**[Table 6]**

| Gr oup | Vaccine composition | IgG1 antibody | | | Averag e | Fold | Log2 averag e | Log2 standard deviation |
|---|---|---|---|---|---|---|---|---|
| 1 | PBS | 0 | 0 | 0 | N/A | N/A | N/A | N/A |
| 2 | Shingrix | 312500 | 312500 | 312500 | 312500 | 1.0 | 18.3 | 0.0 |
| 3 | gE-Fc (10 µg)+ MPL + alum | 156250 0 | 156250 0 | 156250 0 | 156250 0 | 5.0 | 20.6 | 0.0 |
| 4 | gE-Fc' (10 µg)+ MPL + alum | 156250 0 | 156250 0 | 156250 0 | 156250 0 | 5.0 | 20.6 | 0.0 |

## Claims

1. A fusion protein comprising a glycoprotein E (gE) of a varicella zoster virus (VZV) and a constant region of an immunoglobulin molecule.

2. The fusion protein of claim 1, wherein the glycoprotein E of the varicella zoster virus is truncated such that a C-terminus anchor region is removed.

3. The fusion protein of claim 2, wherein the glycoprotein E of the varicella zoster virus has the sequence of SEQ ID NO: 1.

4. The fusion protein of claim 1, wherein the immunoglobulin is any one selected from the group consisting of IgG, IgM, IgA, IgD, and IgA.

5. The fusion protein of claim 1, wherein the immunoglobulin is IgG.

6. The fusion protein of claim 1, wherein the IgG is any one selected from the group consisting of IgG1, IgG2, IgG3, and IgG4.

7. The fusion protein of claim 1, wherein the constant region of the immunoglobulin molecule is a constant region of an IgG1 heavy chain.

8. The fusion protein of claim 1, wherein the constant region of the immunoglobulin molecule comprises a hinge region, a CH2 domain, and a CH3 domain of IgG1.

9. The fusion protein of claim 1, wherein the constant region of the immunoglobulin molecule further comprises a CH1 domain of IgG1.

10. The fusion protein of claim 1, wherein the constant region of the immunoglobulin molecule comprises a Fc site.

11. The fusion protein of claim 10, wherein the Fc site binds with an Fc receptor to enhance immunogenicity.

12. The fusion protein of claim 10, wherein the Fc site comprises an Fc variant.

13. The fusion protein of claim 12, wherein the Fc variant comprises at least one variant selected from the group consisting of G236A, S239D, A330L, and I332E.

14. The fusion protein of claim 12, wherein the Fc variant comprises G23 6A/S23 9D/A330L/I332E variants.

15. The fusion protein of claim 10, wherein the Fc site comprises the sequence of SEQ ID NO: 2 or the sequence of SEQ ID NO: 3.

16. The fusion protein of claim 12, wherein the Fc variant comprises the sequence of SEQ ID NO: 4 or the sequence of SEQ ID NO: 5.

17. A nucleic acid molecule encoding the fusion protein of any one of claims 1 to 16.

18. An expression vector comprising the nucleic acid molecule of claim 17.

19. A host cell into which the expression vector of claim 18 is transformed.

20. A method of producing a fusion protein, the method comprising:
i) introducing the expression vector of claim 18 into an animal cell expression system; and
ii) performing expression of a fusion protein.

21. An immunogenic composition comprising the fusion protein of any one of claims 1 to 16.

22. The immunogenic composition of claim 21, wherein the fusion protein is a monomer.

23. The immunogenic composition of claim 21, wherein the fusion protein is a dimer.

24. The immunogenic composition of claim 23, wherein the dimer is made by a disulfide bond in a hinge region between two fusion proteins.

25. The immunogenic composition of claim 21, further comprising an adjuvant.

26. The immunogenic composition of claim 25, wherein the adjuvant is any one or more selected from the group consisting of a toll-like receptor 4 (TLR4) agonist, an aluminum salt, a saponin, and a liposome.

27. The immunogenic composition of claim 26, wherein the TLR4 agonist is any one or more selected from the group consisting of monophosphoryl lipid A (MPL) and 3D-MPL.

28. The immunogenic composition of claim 26, wherein the aluminum salt is any one or more selected from the group consisting of aluminum hydroxide, aluminum phosphate, and aluminum sulphate.

29. The immunogenic composition of claim 26, wherein the saponin is any one or more selected from the group consisting of QS21, QS17, and QuilA.

30. A kit comprising the immunogenic composition of any one of claims 21 to 29.

31. A method of eliciting an immune response by administering the immunogenic composition of any one of claims 21 to 29.

32. A method of preventing varicella zoster or postherpetic neuralgia by administering the immunogenic composition of any one of claims 21 to 29.
